# EUROPÄISCHE PATENTANMELDUNG

(11) **EP 0 619 307 A1**
(43) Veröffentlichungstag der Anmeldung: **12.10.1994**
(21) Anmeldenummer: 94104674.0
(22) Anmeldetag: 24.03.1994
(51) Int. Cl.: C07D 213/61, C07D 213/85

(54) **Verfahren zur Herstellung von 2-Halogen-pyridin-derivaten**

(30) Priorität: 06.04.1993 DE 4311247
(71) Anmelder: BAYER AG, 51368 Leverkusen (DE)
(72) Erfinder: Rivadeneira, Eric, Dr., D-51381 Leverkusen (DE); Jelich, Klaus, Dr., D-42113 Wuppertal (DE)

(57) **Zusammenfassung**

Die vorliegende Erfindung betrifft ein Verfahren zur Herstellung von 2-Halogen-pyridin-derivaten der allgemeinen Formel (I) 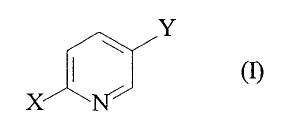
in welcher
- X: für Halogen steht und
- Y: für Halogen, Nitro, Formyl, Cyano, Carboxy, Carbamoyl, Alkyl, Halogenalkyl, Alkoxyalkyl, Dialkoxyalkyl, Alkoxycarbonyl, Alkylaminocarbonyl oder Dialkylaminocarbonyl steht,
ausgenommen Verbindungen, in denen X für Chlor und Y gleichzeitig für Methyl steht,
das dadurch gekennzeichnet ist, daß man
in einer ersten Stufe Pyridin-1-oxide der allgemeinen Formel (II) 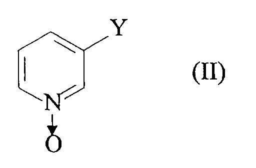
in welcher
- Y: die oben angegebene Bedeutung hat,
mit einer organischen Stickstoffbase A und einer elektrophilen Verbindung, gegebenenfalls in Gegenwart eines Verdünnungsmittels, zu Verbindungen der allgemeinen Formel (III) umsetzt 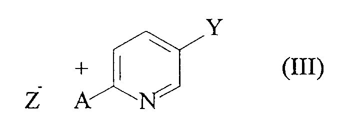
in welcher
- A: für den Rest einer organischen Stickstoffbase steht,
- Y: die oben angegebene Bedeutung hat und
- Z⁻: für ein aus einer elektrophilen Verbindung gebildetes Anion steht,
die Verbindungen der Formel (III) gegebenenfalls als Rohprodukte isoliert oder gegebenenfalls weiter reinigt
und in einer zweiten Stufe mit einem Halogenierungsmittel, gegebenenfalls in Gegenwart eines Alkylhalogenids und gegebenenfalls in Gegenwart eines Verdünnungsmittels bei Temperaturen zwischen 10°C und 150°C umsetzt. Ferner betrifft die Erfindung die neuen Verbindungen der Formel (III).

## Beschreibung

Die Erfindung betrifft ein neues Verfahren zur Herstellung von 2-Halogen-pyridin-derivaten und neue Zwischenprodukte hierfür.

Es ist bekannt, daß man 2-Halogen-pyridin-derivate erhält, wenn man entsprechende Pyridln-1-oxide mit Phosphorylchlorid (vgl. Chem. Pharm. Bull. 36 (1988), 2244-2247; EP-A 324174/LeA 25745), mit chlorhaltigen Phosphorsäurederivaten (vgl. EP-A 370317/LeA 26429), mit Carbonsäurechloriden (vgl. EP-A 438691/LeA 27429), mit Sulfonsäurechloriden (vgl. EP-A 463464/Le A 27658) oder mit anderen Chlorierungsmitteln (vgl. EP-A 439745/LeA 27472), gegebenenfalls in Gegenwart von basischen organischen Stickstoffverbindungen umsetzt.

Die bei diesen Verfahren erzielten Ausbeuten und die Qualität der hierbei erhaltenen Produkte, welche oft erhebliche Mengen an Isomeren enthalten, sind jedoch in vielen Fällen nicht zufriedenstellend.

Ein Verfahren zur Herstellung von 2-Chlor-5-methyl-pyridin, bei welchem 3-Methyl-pyridin-1-oxid zunächst mit einer organischen Stickstoffbase zu einem isolierbaren 1:1-Addukt und letzteres mit einem Chlorierungsmittel umgesetzt wird, ist Gegenstand einer vorgängigen, jedoch nicht vorveröffentlichten Patentanmeldung (vgl. DE-P 4204920/LeA 28939).

Es wurde nun gefunden, daß man 2-Halogen-pyridin-derivate der allgemeinen Formel (I) 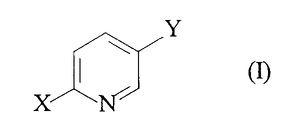
in welcher
- X: für Halogen steht und
- Y: für Halogen, Nitro, Formyl, Cyano, Carboxy, Carbamoyl, Alkyl, Halogenalkyl, Alkoxyalkyl, Dialkoxyalkyl, Alkoxycarbonyl, Alkylaminocarbonyl oder Dialkylaminocarbonyl steht,
ausgenommen Verbindungen, in denen X für Chlor und Y gleichzeitig für Methyl steht,
in guten Ausbeuten und in guter Qualität erhalten kann, wenn man
in einer ersten Stufe Pyridin-1-oxide der allgemeinen Formel (II) 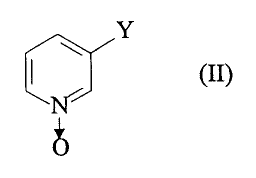
in welcher
- Y: die oben angegebene Bedeutung hat,
mit einer organischen Stickstoffbase A und einer elektrophilen Verbindung, gegebenenfalls in Gegenwart eines Verdünnungsmittels, zu Verbindungen der allgemeinen Formel (III) umsetzt 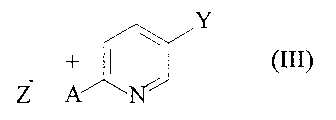
in welcher
- A: für den Rest einer organischen Stickstoffbase steht,
- Y: die oben angegebene Bedeutung hat und
- Z⁻: für ein aus einer elektrophilen Verbindung gebildetes Anion steht,
die Verbindungen der Formel (III) gegebenenfalls als Rohprodukte isoliert oder gegebenenfalls weiter reinigt
und in einer zweiten Stufe mit einem Halogenierungsmittel, gegebenenfalls in Gegenwart eines Alkylhalogenids und gegebenenfalls in Gegenwart eines Verdünnungsmittels bei Temperaturen zwischen 10°C und 150°C umsetzt.

Überraschenderweise könne nah dem erfindungsgemäßen Verfahren 2-Halogen-pyridin-derivate der Formel (I) in hohen Ausbeuten und in erheblich verbesserter Isomerenreinheit (im Vergleich mit der bekannten Verfahrensweise) erhalten werden. Nicht umgesetzte Zwischenprodukte der Formel (III) können erneut in den Prozeß eingesetzt werden.

Das erfindungsgemäße Verfahren stellt somit eine wertvolle Bereicherung des Standes der Technik dar.

Das erfindungsgemäße Verfahren betrifft vorzugsweise die Herstellung von Verbindungen der Formel (I), in welcher
- X: für Fluor, Chlor, Brom oder Iod steht und
- Y: für Fluor, Chlor, Brom, Iod, Nitro, Formyl, Cyano, Carboxy, Carbamoyl, für gegebenenfalls durch Fluor, Chlor, Brom (einfach bis dreifach) oder durch C₁-C₄-Alkoxy (einfach oder zweifach) substituiertes C₁-C₄-Alkyl, oder für C₁-C₄-Alkoxy-carbonyl, C₁₋C₄-Alkylamino-carbonyl oder Di-(C₁-C₄-alkyl)-amino-carbonyl steht.

Das erfindungsgemäße Verfahren betrifft insbesondere die Herstellung von Verbindungen der Formel (I), in welcher
- X: für Fluor, Chlor oder Brom steht und
- Y: für Fluor, Chlor, Brom, Formyl, Cyano, Carboxy, Carbamoyl, Ethyl, n- oder i-Propyl, n-, i-, s- oder t-Butyl, Trifluormethyl, Trichlormethyl, Tribrommethyl, Difluormethyl, Dichlormethyl, Dibrommethyl, Fluormethyl, Chlormethyl, Brommethyl, Methoxymethyl, Ethoxymethyl, n- oder i-Propoxymethyl, n-, i-, s- oder t-Butoxymethyl, Dimethoxymethyl oder Diethoxymethyl, Methoxycarbonyl, Ethoxycarbonyl, Methylaminocarbonyl, Ethylaminocarbonyl, Dimethylaminocarbonyl oder Diethylaminocarbonyl steht.

Verwendet man beispielsweise 3-Fluor-pyridin-1-oxid als Ausgangsstoff, Trimethylamin als organische Stickstoffbase A und Phosgen als elektrophile Verbindung in der ersten Verfahrensstufe und Phosgen als Halogenierungsmittel in der zweiten Verfahrensstufe, so kann der Reaktionsablauf beim erfindungsgemäßen Verfahren durch das folgende Formelschema skizziert werden: 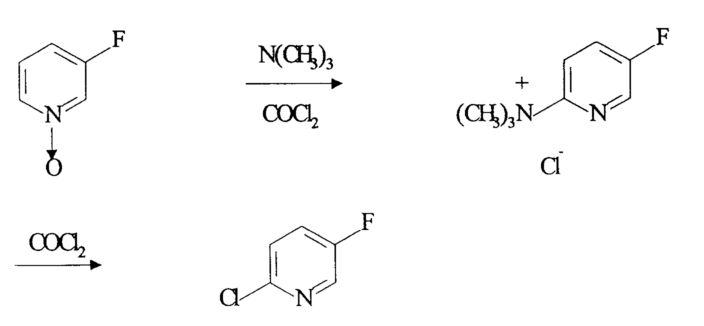
Die beim erfindungsgemäßen Verfahren zur Herstellung von Verbindungen der Formel (I) als Ausgangsstoffe zu verwendenden Pyridin-1-oxide sind durch die Formel (II) allgemein definiert.

In Formel (II) hat Y vorzugsweise bzw. insbesondere diejenige Bedeutung, die bereits oben im Zusammenhang mit der Beschreibung der erfindungsgemäß herzustellenden Verbindungen der Formel (I) vorzugsweise bzw. als insbesondere bevorzugt für Y angegeben wurde.

Die Ausgangsstoffe der Formel (II) sind bekannte organische Chemikalien (vgl. J. Chem. Soc. 1959, 3680-3683; J. Med. Chem. 11 (1968), 1172-1176; Liebigs Ann. Chem. 618 (1958), 152-158; Z. Chem. 10 (1970), 184-185).

Die in der ersten Stufe des erfindungsgemäßen Verfahrens gebildeten Addukte sind durch die Formel (III) allgemein definiert.

In der Formel (III) stehen vorzugsweise
- A: für eine organische Stickstoffbase aus der Reihe Tri-(C₁-C₄-alkyl)-amin, N,N-Di-(C₁-C₄-alkyl)-benzylamin, N,N-Di-(C₁-C₄-alkyl)-cyclohexylamin oder für Pyridin, welches gegebenenfalls einfach bis dreifach durch C₁-C₄-Alkyl substituiert ist, und
- Z⁻: für ein Chlorid- oder ein C₁-C₄-Alkyl-carboxylat-ion;
- Y: hat vorzugsweise diejenige Bedeutung, die oben im Zusammenhang mit der Beschreibung der erfindungsgemäß herzustellenden Verbindungen der Formel (I) als bevorzugt angegeben wurde.

Insbesondere stehen in Formel (III)
- A: für Trimethylamin, Triethylamin, Tripropylamin, Tributylamin, N,N-Dimethyl-benzylamin, N,N-Diethylbenzylamin, N,N-Dimethyl-cyclohexylamin, N,N-Diethyl-cyclohexylamin oder Pyridin, welches gegebenenfalls einfach oder zweifach durch Methyl oder Ethyl substituiert ist, und
- Z⁻: für ein Chlorid- oder ein Acetat-ion;
- Y: hat insbesondere diejenige Bedeutung, die oben im Zusammenhang mit der Beschreibung der erfindungsgemäß herzustellenden Verbindungen der Formel (I) als insbesondere bevorzugt angegeben wurde.

Die Addukte der Formel (III) sind noch nicht aus der Literatur bekannt und mit Ausnahme von Trimethyl-(5-methyl-pyridin-2-yl)-ammoniumchlorid und Triethyl-(5-methyl-pyridin-2-yl)-ammoniumchlorid - vgl. die vorgängige Anmeldung DE-P 4204920 - als neue chemische Verbindungen Gegenstand der vorliegenden Patentanmeldung.

Das erfindungsgemäße Verfahren wird in der ersten Stufe unter Verwendung einer organischen Stickstoffbase A durchgeführt. Die bevorzugte bzw. die insbesondere bevorzugte Bedeutung von A ist der Definition der Verbindungen der Formel (III) zu entnehmen.

Trimethylamin wird als organische Stickstoffbase für das erfindungsgemäße Verfahren ganz besonders bevorzugt.

In der ersten Stufe des erfindungsgemäßen Verfahrens wird weiterhin eine elektrophile Verbindung eingesetzt. Bevorzugte elektrophile Verbindungen für das erfindungsgemäße Verfahren sind Säurechloride und Anhydride, beispielsweise Acetylchlorid, Propionylchlorid, Acetanhydrid, Propionsäureanhydrid, Benzoylchlorid, Benzotrichlorid, Phosgen, Oxalylchlorid, Benzolsulfochlorid, p-Toluolsulfonsäurechlorid, Phosphor(III)-chlorid, Phosphorylchlorid (Phosphoroxychlorid), Phosphor(V)-chlorid, Thionylchlorid, Sulfurylchlorid, Dichlormethylen-dimethylimmoniumchlorid, Cyanurchlorid und Chlortrimethylsilan.

Phosgen, Thionylchlorid, Sulfurylchlorid, Benzolsulfonsäurechlorid und p-Toluolsulfonsäurechlorid werden als elektrophile Einsatzstoffe für das erfindungsgemäße Verfahren besonders bevorzugt.

Das erfindungsgemäße Verfahren wird in der zweiten Stufe unter Verwendung eines Halogeniermittels durchgeführt. Bevorzugte Halogenierungsmittel sind beim erfindungsgemäßen Verfahren Verbindungen, welche Halogenionen an andere Reaktionspartner abgeben können, beispielsweise Acetylchlorid, Benzoylchlorid, Phosgen, Oxalylchlorid, Benzolsulfochlorid, Phosphor(III)-chlorid, Phosphor(III)-bromid, Phosphorylchlorid, Phosphor(V)-chlorid, Thionylchlorid, Sulfurylchlorid, Hydrogenchlorid, Hydrogenfluorid, Hydrogenbromid, Tetraethylammoniumchlorid, Tetrabutylammoniumchlorid und Benzyl-triethylammoniumchlorid.

Phosgen, Hydrogenfluorid, Hydrogenchlorid und Hydrogenbromid werden als Halogenierungsmittel in der zweiten Stufe des erfindungsgemäßen Verfahrens besonders bevorzugt.

Das erfindungsgemäße Verfahren wird in der zweiten Stufe gegebenenfalls in Gegenwart eines Alkylhalogenids durchgeführt. Bevorzugte Alkylchloride sind Methylchlorid, Methylbromid, Ethylchlorid, Propylchlorid und Butylchlorid, insbesondere Methylchlorid.

Das erfindungsgemäße Verfahren wird in der ersten Stufe vorzugsweise unter Verwendung eines Verdünnungsmittels durchgeführt. Als Verdünnungsmittel kommen praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofüran und Dioxan, Ketone wie Aceton, Methylethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuretriamid.

Das erfindungsgemäße Verfahren wird gegebenenfalls auch in der zweiten Stufe in Gegenwart eines Verdünnungsmittels durchgeführt. Als Verdünnungsmittel kommen hierbei neben Wasser praktisch alle inerten organischen Lösungsmittel in Betracht. Hierzu gehören vorzugsweise Alkohole wie Methanol, Ethanol, n- und i-Propanol, Carbonsäuren wie Ameisensäure, Essigsäure und Propionsäure, sowie die oben für die erste Stufe des erfindungsgemäßen Verfahrens angegebenen Solventien.

Die erste Stufe des erfindungsgemäßen Verfahrens wird falls Trimethylamin als organische Stickstoffbase eingesetzt wird, im allgemeinen im Temperaturbereich zwischen -50°C und +120°C, vorzugsweise zwischen -30°C und +80°C durchgeführt. Falls andere organische Stickstoffbasen als Trimethylamin eingesetzt werden, wird im allgemeinen im Temperaturbereich von -30°C bis +100°C, vorzugsweise zwischen -15°C und +10°C gearbeitet.

Die erste Stufe des erfindungsgemäßen Verfahrens wird im allgemeinen bei Normaldruck durchgeführt. Es ist jedoch auch möglich, unter erhöhtem oder vermindertem Druck, im allgemeinen zwischen 0,1 und 100 bar, zu arbeiten.

Zur Durchführung der ersten Stufe des erfindungsgemäßen Verfahrens setzt man auf 1 Mol Ausgangsverbindung der Formel (II) im allgemeinen zwischen 1 und 10 Mol, vorzugsweise zwischen 1 und 5 Mol, organische Slickstoffbase und zwischen 1 und 10 Mol, vorzugsweise zwischen 1 und 5 Mol, elektrophile Verbindung ein.

In einer bevorzugten Ausführungsform der ersten Stufe des erfindungsgemäßen Verfahrens wird die Ausgangsverbindung der Formel (II) in einem Verdünnungsmittel vorgelegt und nach Abkühlen werden nacheinander zuerst die organische Stickstoffbase und dann die elektrophile Verbindung langsam unter Rühren eindosiert.

Nach dem Ende der Umsetzung werden gegebenenfalls die leichter flüchtigen Komponenten unter vermindertem Druck abdestilliert. Das Roh-Zwischenprodukt, welches im wesentlichen das Addukt der Formel (III) enthält, kann auf übliche Weise, z.B. durch Säulenchromatographie gereinigt oder auch roh in die zweite Stufe eingesetzt werden.

Vorzugsweise wird das Roh-Zwischenprodukt als solches - gegebenenfalls auch ohne Entfernen des Verdünnungsmittels - in die zweite Stufe eingesetzt.

Die zweite Stufe des erfindungsgemäßen Verfahrens wird im allgemeinen im Temperaturbereich zwischen 10°C und 150°C, vorzugsweise zwischen 20°C und 120°C, insbesondere zwischen 30°C und 1 10°C, durchgeführt.

Die zweite Stufe des erfindungsgemäßen Verfahrens wird im allgemeinen bei Normaldruck durchgeführt. Es ist jedoch auch möglich, unter erhöhtem oder vermindertem Druck, im allgemeinen zwischen 0,01 und 200 bar, zu arbeiten.

Die Aufarbeitung kann auf übliche Weise durchgeführt werden. Beispielsweise wird die Rohsubstanz mit einem mit Wasser praktisch nicht mischbaren organischen Lösungsmittel digeriert, mit Wasser gewaschen und getrocknet. Nach sorgfältigem Abdestillieren des organischen Lösungsmittel unter vermindertem Druck verbleibt ein Rückstand, welcher im wesentlichen das Produkt der Formel (I) enthält.

Die nach dem erfindungsgemäßen Verfahren herstellbaren 2-Halogen-pyridin-Derivate können als Zwischenprodukte zur Herstellung von Pharmazeutika (vgl. DE-A 2812585) oder von Pflanzenbehandlungsmitteln (vgl. DE-A 2630046, EP-A 192060, DE-A 3726993, DE-A 3924682) eingesetzt werden.

### Herstellungsbeispiele:

### Beispiel 1

### 1. Stufe:

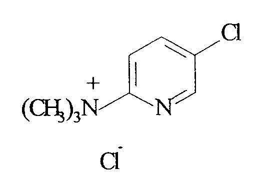

47,2 g (0,8 Mol) Trimethylamin werden bei -25°C in eine Lösung von 32,4 g (0,25 Mol) 3-Chlorpyridin-N-oxid in 250 ml 1,2-Dichlorethan einkondensiert. Innerhalb von 40 Minuten werden bei 0°C 52,95 g (0,31 Mol) Benzolsulfonsäureclalorid eingetropft. Man läßt die Mischung 2 Stunden bei 0°C rühren und anschließend über Nacht auftauen. Das Gemisch wird im Vakuum bei einer Badtemperatur von unter 50°C eingeengt und durch wiederholte Säulenchromatographie gereinigt.

Man erhält 24,3 g (47% der Theorie) Trimethyl-2-(5-chlorpyridyl)-ammoniumchlorid.
- ¹H-NMR (300 MHz, D₆-DMSO):: δ/ppm = 3,63 (9H, Singulett), 8,25 (IH, Dublett, J = 8,7 Hz), 8,42 (IH, Dublett von Dublett, J₁ = 9 Hz, J₂ = 2,4 Hz, 8,77 (IH, Dublett, J = 2,4 Hz).

### 2. Stufe:

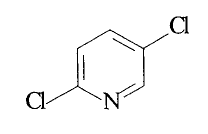

Ausgehend von 32,4 g (0,25 Mol) 3-Chlorpyridin-N-oxid wird nach obiger Vorschrift das Trimethyl-2-(5-chlorpyridyl)-ammoniumchlorid hergestellt, allerdings ohne säulenchromatographische Reinigung des Rohprodukts. Das Rohprodukt wird in 500 ml 1,2-Dichlorethan gegeben und bei 50°C 30 Stunden lang mit gasförmigem Chlorwasserstoff versetzt. Anschließend wird eingeengt, in Wasser aufgenommen, mit verdünnter Natronlauge auf pH 9-10 gestellt und dreimal mit Methylenchlorid extrahiert. Die vereinigten organischen Phasen werden über Natriumsulfat getrocknet, filtriert und am Rotationsverdampfer eingeengt. Das Rohprodukt wird anschließend säulenchromatographisch gereinigt.

Man erhält 19,7 g (53% der Theorie bezogen auf eingesetztes 3-Chlorpyridin-N-oxid) 2,5-Dichlorpyridin.

### Beispiel 2

### 1. Stufe:

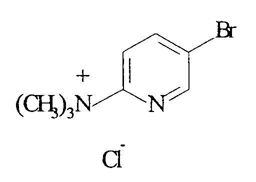

41,9 g (0,71 Mol) Trimethylamin werden bei -25°C in eine Lösung von 37,4 g (0,215 Mol) 3-Brompyridin-N-oxid in 250 ml 1,2-Dichlorethan einkondensiert. Innerhalb einer Stunde werden bei 0°C 45,6 g (0,258 Mol) Benzolsulfonsäurechlorid eingetropft. Man rührt 2 Stunden bei 0°C nach und läßt dann über Nacht auftauen. Das Gemisch wird im Vakuum bei einer Badtemperatur von unter 50°C eingeengt und durch mehrfache Säulenchromatographie gereinigt.

Man erhält 27,4 g (50,7% der Theorie) Trimethyl-2-(5-brompyridyl)-ammoniumchlorid.
- ¹H-NMR (300 MHz, D₆-DMSO):: δ/ppm = 3,61 (9H, Singulett), 8,145 (IH, Dublett, J = 8,7 Hz), 8,54 (IH, Dublett von Dublett, J₁ = 9Hz, J₂ = 2,4 Hz), 8,85 (IH, Dublett, J = 2,4 Hz).

### 2. Stufe:

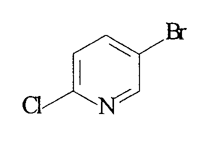

Ausgehend von 52,2 g (0,3 Mol) 3-Brompyridin-N-oxid wird nach obiger Vorschrift das Trimethyl-2-(5-brompyridyl)-ammoniumchlorid hergestellt, allerdings ohne säulenchromatographische Reinigung des Rohprodukts. Das Rohprodukt wird in 500 ml 1,2-Dichlorethan gegeben und bei 50°C 30 Stunden lang mit gasförmigem Chlorwasserstoff versetzt. Anschließend wird eingeengt, in Wasser aufgenommen, mit verdünnter Natronlauge alkalisch gestellt (pH 9-10) und dreimal mit Methylenchlorid extrahiert. Die vereinigten organischen Phasen werden über Natriumsulfat getrocknet, filtriert und am Rotationsverdampfer eingeengt. Das Rohprodukt wird säulenchromatographisch gereinigt.

Man erhält 32,7 g (56,6% der Theorie) bezogen auf eingesetztes 3-Brompyridin-N-oxid) 5-Brom-2-chlorpyridin.

### Beispiel 3:

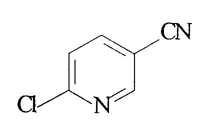

6 g (0,03 Mol) Trimethyl-2-(5-cyanopyridyl)-ammoniumchlorid werden in 80 ml 1,2-Dichlorethan gegeben und bei 50°C 5 Stunden lang mit gasförmigen Chlorwasserstoff versetzt. Anschließend wird eingeengt, in Wasser aufgenommen, mit Natronlauge auf pH 9-10 eingestellt und dreimal mit Methylenchlorid extrahiert. Die vereinigten organischen Phasen werden über Natriumsulfat getrocknet, filtriert und am Rotationsverdampfer eingeengt.

Man erhält 3,8 g (91% der Theorie) 2-Chlor-5-cyanopyridin.

### Beispiel 4:

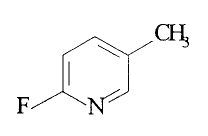

54,5 g (0,5 Mol) 3-Methylpyridin-1-oxid werden in 350 ml Methylenchlorid vorgelegt und das Gemisch wird auf 0°C abgekühlt. Bei dieser Temperatur werden 202 g (2 Mol) Trimethylamin einkondensiert. Dann werden 148,5 g (1,6 Mol) Phosgen eingeleitet, wobei die Temperatur durch intensives Kühlen bei 0°C gehalten wird. Nach Beendigung der Reaktion wird überschüssiges Phosgen bei 20°C im Wasserstrahlvakuum entfernt. Der verbleibende Rückstand wird in 350 ml 1,2-Dichlorethan gelöst und mit 30 g (1,5 Mol) wasserfreiem Fluorwasserstoff versetzt. Die Apparatur wird verschlossen, mit einem Stickstoff-Druck von 10 bar versehen und auf 120°C erhitzt. Bei dieser Temperatur wird 20 Stunden lang gerührt. Anschließend läßt man abkühlen, entfernt den überschüssigen Fluorwasserstoff im Wasserstrahlvakuum und gibt den Rückstand auf Eis. Mit festem Kaliumhydroxid wird alkalisch gestellt und anschließend mit Methylenchlorid extrahiert. Nach Trocknen und Einengen der organischen Phase wird der Rückstand destilliert.

Man erhält 38 g (68,5 % der Theorie) 2-Fluor-5-methylpyridin (Kp₄₈₋₄₅ = 65-75°C).

## Patentansprüche

1. Verfahren zur Herstellung von 2-Halogen-pyridin-derivaten der allgemeinen Formel (I) 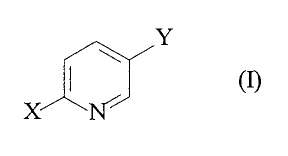 in welcher
X für Halogen steht und
Y für Halogen, Nitro, Formyl, Cyano, Carboxy, Carbamoyl, Alkyl, Halogenalkyl, Alkoxyalkyl, Dialkoxyalkyl, Alkoxycarbonyl, Alkylaminocarbonyl oder Dialkylaminocarbonyl steht,
ausgenommen Verbindungen, in denen X für Chlor und Y gleichzeitig für Methyl steht,
dadurch gekennzeichnet, daß man in einer ersten Stufe Pyridin-1-oxide der allgemeinen Formel (II) 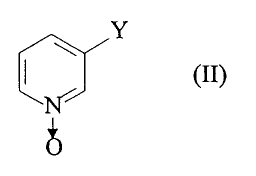 in welcher
Y die oben angegebene Bedeutung hat,
mit einer organischen Stickstoffbase A und einer elektrophilen Verbindung, gegebenenfalls in Gegenwart eines Verdünnungsmittels, zu Verbindungen der allgemeinen Formel (III) umsetzt 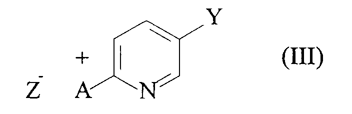 in welcher
A für den Rest einer organischen Stickstoffbase steht,
Y die oben angegebene Bedeutung hat und
Z⁻ für ein aus einer elektrophilen Verbindung gebildetes Anion steht,
die Verbindungen der Formel (III) gegebenenfalls als Rohprodukte isoliert oder gegebenenfalls weiter reinigt
und in einer zweiten Stufe mit einem Halogenierungsmittel, gegebenenfalls in Gegenwart eines Alkylhalogenids und gegebenenfalls in Gegenwart eines Verdünnungsmittels bei Temperaturen zwischen 10°C und 150°C umsetzt.

2. Verfahren gemäß Anspruch 1, dadurch gekennzeichnet, daß
X für Fluor, Chlor, Brom oder Iod steht und
Y für Fluor, Chlor, Brom, Iod, Nitro, Formyl, Cyano, Carboxy, Carbamoyl, für gegebenenfalls durch Fluor, Chlor, Brom (einfach bis dreifach) oder durch C₁-C₄-Alkoxy (einfach oder zweifach) substituiertes C₁-C₄-Alkyl, oder für C₁-C₄-Alkoxy-carbonyl, C₁-C₄-Alkylamino-carbonyl oder Di-(C₁-C₄-alkyl)-amino-carbonyl steht mit Ausnahme von Verbindungen in denen X für Chlor und Y gleichzeitig für Methyl steht.

3. Verfahren gemäß Anspruch 1, dadurch gekennzeichnet, daß
X für Fluor, Chlor oder Brom steht und
Y für Fluor, Chlor, Brom, Formyl, Cyano, Carboxy, Carbamoyl, Ethyl, n- oder i-Propyl, n-, i-, s- oder t-Butyl, Trifluormethyl, Trichlormethyl, Tribrommethyl, Difluormethyl, Dichlormethyl, Dibrommethyl, Fluormethyl, Chlormethyl, Brommethyl, Methoxymethyl, Ethoxymethyl, n- oder i-Propoxymethyl, n-, i-, s- oder t-Butoxymethyl, Dimethoxymethyl oder Diethoxymethyl, Methoxycarbonyl, Ethoxycarbonyl, Methylaminocarbonyl, Ethylaminocarbonyl, Dimethylaminocarbonyl oder Diethylaminocarbonyl steht.

4. Verbindungen der Formel (III) 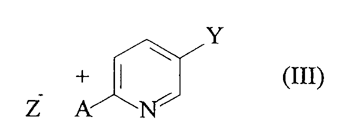 in welcher
A für den kationischen Rest einer organischen Stickstoffbase steht,
Z für ein an einer elektrophilen Verbindung gebildetes Anion steht,
Y für Halogen, Nitro, Formyl, Cyano, Carboxy, Carbamoyl, Alkyl, Halogenalkyl, Alkoxyalkyl, Dialkoxyalkyl, Alkoxycarbonyl, Alkylaminocarbonyl oder Dialkylaminocarbonyl steht,
mit Ausnahme von Trimethyl-(5-methyl-pyridin-2-yl)-ammoniumchlorid und Triethyl-(5-methyl-pyridin-2-yl)-ammoniumchlorid.

5. Verbindungen der Formel (III) gemäß Anspruch 4 wobei
A für eine organische Stickstoffbase aus der Reihe Tri-(C₁-C₄-alkyl)-amin, N,N-Di-(C₁-C₄-alkyl)-benzylamin, N,N-Di-(C₁-C₄-alkyl)-cyclohexylamin oder für Pyridin, welches gegebenenfalls einfach bis dreifach durch C₁-C₄-Alkyl substituiert ist, und
Z⁻ für ein Chlorid- oder ein C₁-C₄-Alkyl-carboxylat-ion;
Y für Fluor, Chlor, Brom, Iod, Nitro, Formyl, Cyano, Carboxy, Carbamoyl, für gegebenenfalls durch Fluor, Chlor, Brom (einfach bis dreifach) oder durch C₁-C₄-Alkoxy (einfach oder zweifach) substituiertes C₁-C₄-Alkyl, oder für C₁-C₄-Alkoxy-carbonyl, C₁-C₄-Alkylamino-carbonyl oder Di-(C₁-C₄-alkyl)-amino-carbonyl steht,
mit Ausnahme von Trimethyl-(5-methyl-pyridin-2-yl)-ammoniumchlorid und Triethyl-(5-methyl-pyridin-2-yl)-ammoniumchlorid.

6. Verbindungen der Formel (III) gemäß Anspruch 4, wobei
A für Trimethylamin, Triethylamin, Tripropylamin, Tributylamin, N,N-Dimethyl-benzylamin, N,N-Diethylbenzylamin, N,N-Dimethyl-cyclohexylamin, N,N-Diethyl-cyclohexylamin oder Pyridin, welches gegebenenfalls einfach oder zweifach durch Methyl oder Ethyl substituiert ist, steht,
Z⁻ für ein Chlorid- oder ein Acetat-ion steht,
Y für Fluor, Chlor, Brom, Formyl, Cyano, Carboxy, Carbamoyl, Ethyl, n- oder i-Propyl, n-, i-, s- oder t-Butyl, Trifluormethyl, Trichlormethyl, Tribrommethyl, Difluormethyl, Dichlormethyl, Dibrommethyl, Fluormethyl, Chlormethyl, Brommethyl, Methoxymethyl, Ethoxymethyl, n- oder i-Propoxymethyl, n-, i-, s- oder t-Butoxymethyl, Dimethoxymethyl oder Diethoxymethyl, Methoxycarbonyl, Ethoxycarbonyl, Methylaminocarbonyl, Ethylaminocarbonyl, Dimethylaminocarbonyl oder Diethylaminocarbonyl steht
mit Ausnahme von Trimethyl-(5-methyl-pyridin-2-yl)-ammoniumchlorid und Triethyl-(5-methyl-pyridin-2-yl)-ammoniumchlorid.
